# EUROPÄISCHE PATENTSCHRIFT

(11) **EP 1 471 831 B1**
(45) Veröffentlichungstag und Bekanntmachung des Hinweises auf die Patenterteilung: **31.08.2005**
(21) Anmeldenummer: 03706449.0
(22) Anmeldetag: 07.02.2003
(51) Int. Cl.: A61B 17/02

(54) **HERZHALTER**
HEART HOLDER
SUPPORT POUR MUSCLE CARDIAQUE

(30) Priorität: 08.02.2002 EP 02002860
(43) Veröffentlichungstag der Anmeldung: 03.11.2004
(73) Patentinhaber: Karl Storz GmbH & Co. KG, 78532 Tuttlingen (DE)
(72) Erfinder: SCHÖLLHORN, Joachim, 79104 Freiburg (DE)
(74) Vertreter: Weller, Wolfgang
(86) Internationale Anmeldenummer: PCT/EP2003/001206
(87) Internationale Veröffentlichungsnummer: WO 2003/065901

(56) Entgegenhaltungen:
- US-A- 6 019 722

## Beschreibung

Die Erfindung betrifft ein Funktionselement zum Anbringen an einen Retraktor zur Durchführung von herz- und thoraxchirurgischen Eingriffen, mit einer Befestigungsvorrichtung zum lösbaren Anbringen an den Retraktor, mit einer Vorrichtung zum Hochhalten eines von einer Operationsstelle angehobenen schlagenden Herzens, die mehrere spreizbare streifenförmige Haltefinger aufweist.

Ein derartiges Funktionselement ist aus der US 6,019,722 bekannt.

Ein Retraktor weist eine Zahnschiene auf, von der eine erste Halteschiene abgewinkelt ist, sowie eine längs der Zahnschiene über einen Antriebsmechanismus verfahrbare zweite Halteschiene auf, die sich parallel zur ersten Halteschiene erstreckt und auf diese zu bzw. von dieser weg bewegbar ist.

An den Halteschienen sind lösbar unterschiedliche Funktionselemente anbringbar.

Retraktoren dieser Art werden beispielsweise als Rippensperrer bei Operationen am offen schlagenden Herzen verwendet. Für solche Retraktoren haben sich auch die Fachbegriffe wie Thorax- oder Sternum-Sperrer etabliert. Bei der Verwendung als Sternum (Brustbein) - Sperrer wird das Brustbein der Länge nach aufgetrennt, und hakenförmige Funktionselemente sowohl an der festen als auch an der beweglichen Halteschiene werden in die Operationsöffnung eingesetzt. Durch Betätigen des Antriebsmechanismus bewegen sich bewegliche und ortsfeste Halteschiene voneinander weg und dabei wird der Brustkorb gespreizt und eine Zugangsöffnung geschaffen, durch die der Operateur insbesondere am schlagenden Herz operative Eingriffe vornehmen kann.

An die Halteschienen können noch weitere Funktionselemente lösbar angebracht werden, um während der Operation den Operateur zu unterstützen.

Solche weitere Funktionselemente sind beispielsweise ein Leyla-Arm, der als Hilfsinstrument beim Vernähen von Blutgefäßen dient. Andere Funktionselemente sind MIDCAB = (Minimal Invasive Direct Coronary Artery Bypass) - Sperrer oder IMA-Sperrer um einen Zugang zur Brustbeinarterie zu erhalten, die dazu dienen, die eine Brusthälfte, in der das Herz vorhanden ist gegenüber der anderen Brusthälfte anzuheben um einen weiter verbesserten Zugang zum Herzen zu erhalten.

Eine Vorrichtung zum Hochhalten eines von einer Operationsstelle angehobenen schlagenden Herzens, die mehrere spreizbare streifenförmige Haltefinger aufweist, ist aus der US 6,019,722 bekannt. Mit dieser Vorrichtung kann ein schlagendes Herz im Bereich dessen Herzspitze vom geöffneten Brustraum aus angehoben und gehalten werden, wodurch ein Zugang zur Herzhinterwand möglich ist, um daran operative Eingriffe vorzunehmen. Da das Herz noch schlägt, besteht das Problem, ein pulsierendes Organ für die Dauer einer Operation in einem angehobenen Zustand hochzuhalten.

Bei der US 6,019,722 weist die Vorrichtung zum Hochhalten zwei Typen an Halteelementen auf. Ein erster Typ, dort rigid cross support genannt, besteht aus einem steifen unbiegsamen Material. In einer ersten Ausführungsform ist dieser erste Typ als eine Art Tasse oder Becher ausgebildet, in der die Herzspitze eingelegt werden kann. Von der Tasse bzw. dem Becher wird das Hauptgewicht des Herzens getragen. Zusätzlich wird das Herz durch Unterdruck in die Tasse bzw. den Becher gesaugt. In einer weiteren Ausführungsform besteht dieser erste Typ aus zwei spreizbaren streifenförmigen steifen Haltefingern, die im Bereich der Herzspitze angelegt werden und sich etwa umfänglich um die Herzspitze erstrecken. Die Herzspitze ist zwischen den steifen Haltefingern eingeklemmt, auch hier sind Saugöffnungen zum Ansaugen des Herzens vorgesehen.

Ferner ist zwingend ein zweiter Typ an Halteelementen vorgesehen, und zwar zumindest jeweils vier weitere elastisch biegsame stangenförmige Elemente, die sich im Wesentlichen in Längsrichtung des Herzens erstrecken. Diese vier Halteelemente des zweiten Typs sind biegsam ausgebildet und weisen an ihrem äußeren Ende jeweils einen Saugnapf auf. Durch Anlegen eines Vakuums und auf Grund der Biegsamkeit können diese vier Halteelemente des zweiten Typs den pulsierenden Kontraktionsbewegungen des schlagenden Herzens folgen. Diese bewegen sich also im Wesentlichen in radialen Richtungen bezüglich der Herzlängsachse gesehen und sind dazu beweglich. Dieser zweite Typ wird in diesem Dokument fine support means genannt. Bei der Ausgestaltung mit den spreizbaren streifenförmigen Haltefingern sind also insgesamt sechs Halteelemente vorhanden, zwei vom ersten Typ und vier vom zweiten Typ, die für ein Hochhalten des schlagenden Herzens sorgen. Das Grundprinzip dieses Herzhalters besteht darin, das Hauptgewicht des Herzens im Bereich dessen Spitze durch den rigid cross support (gross weight support means) zu tragen und zusätzlich durch Unterdruck zu immobilisieren. Die elastischen fine support means dienen zur seitlichen Halterung und folgen den Kontraktionsbewegungen des Herzens.

Dadurch, dass eine solche große Anzahl an Halteelementen, nämlich sechs vorhanden sind, müssen zahlreiche Anlagepunkte und Haltepunkte an der äußeren Oberfläche des Herzens belegt werden, wodurch aber zwangsläufig der Zugang zu dem Herzen beschränkt oder behindert ist. Der Sinn und Zweck des Hochhebens ist ja gerade, an der Außenseite des schlagenden Herzens, insbesondere der Herzhinterseite, Zugang zu finden und dort einen chirurgischen Eingriff vorzunehmen. Durch den Käfig von insgesamt sechs Haltefingern ist dieser Zugang doch erheblich behindert.

Ferner ist die Konstruktion äußerst aufwändig und verlangt zwingend die Herstellung und die Handhabung von zwei völlig unterschiedlichen Typen an Halteelementen.

Es ist daher Aufgabe der vorliegenden Erfindung, ein Funktionselement dieser Art zu schaffen, das wesentlich einfacher ausgebildet ist, einfacher zu handhaben ist und den Bereich des gehaltenen Herzens weniger behindert, dennoch aber ein sicheres Hochhalten gewährleistet.

Erfindungsgemäß wird die Aufgabe dadurch gelöst, dass zumindest drei streifenförmige Haltefinger vorhanden sind, die derart biegsam ausgebildet sind, dass diese zu einem an das jeweils zu haltende Herz angepassten Haltekorb formbar sind.

Es wurde festgestellt, dass mit einem einzigen Typ an Halteelementen es möglich ist, ein schlagendes Herz sicher zu halten, und zwar wenn dies mit drei spreizbaren streifenförmigen Haltefingern erfolgt, die aus einem Material hergestellt sind, das derart biegsam ausgebildet ist, dass jeder Streifen an das jeweils zu haltende Herz angepasst werden kann.

Dadurch kann aus diesen drei Streifen ein Haltekorb an Ort und Stelle geformt werden, in dem das schlagende Herz aufgenommen werden kann und sicher gehalten werden kann. Durch die Spreizbarkeit, die an sich bekannt ist, können die drei Streifen so aufgefächert und an das Herz angelegt werden, dass dies seitlich nicht zwischen den Haltefingern herausgleitet. Auf Grund der Biegsamkeit können die Streifen an die jeweilige Anatomie des anzuhebenden Herzens bzw. der Herzspitze angepasst werden.

Insbesondere bei Herzkranken, an denen ja solche Operationen vorgenommen werden, sind pathologische Ausformungen des Herzens vorhanden, so dass durch die Biegsamkeit jeweils an die individuellen Gegebenheiten der Herzform angepasst werden kann. Dadurch kann ein individueller Haltekorb geformt werden, der das angehobene Herz sicher hochhält und dennoch die pulsierenden Bewegungen des Herzens innerhalb des geformten Haltekorbs erlaubt. Ein aus lediglich drei Fingern ausgebildeter Korb lässt drei in sich geschlossene unbehinderte Bereiche dem Operateur frei zugänglich. Dabei sind insbesondere die Bereiche der äußeren Hauptherzkranzgefäße frei, an denen die Operationen vorgenommen werden sollen. Anders ausgedrückt, wenn der Operateur sich einmal den Korb aus den drei Fingern zurechtgeformt hat, sind keine weiteren Änderungen im Laufe der Operation notwendig.

Das Herz liegt in dem Korb, ohne dass es geklemmt ist.

In einer weiteren Ausgestaltung der Erfindung sind die Haltefinger in einer bestimmten Position feststellbar.

Diese an sich bekannte Maßnahme hat den Vorteil, dass der Operateur, nach Formen des Korbes und nach Finden der idealen Position, durch Feststellen der Haltefinger diese Relativlage während des operativen Eingriffes ausreichend fixieren kann.

In einer weiteren Ausgestaltung der Erfindung weisen die Haltefinger auf der dem Herzen zugewandten Seite eine gleithemmende Oberflächenstruktur auf.

Diese Maßnahme hat den Vorteil, dass durch diese gleithemmende Struktur zusätzliche Maßnahmen ergriffen sind, um ein Herausgleiten des Herzens aus dem Korb zu verhindern. Dadurch werden aber auch Drehbewegungen des Herzens in dem Korb unter Lageveränderungen vermieden oder zumindest stark gehemmt. Beim Schlagen des Herzens findet ja eine Kontraktion in radialer Richtung statt, die nach wie vor ungehindert möglich ist.

In einer weiteren Ausgestaltung der Erfindung weist die Oberflächenstruktur pyramidenartige Erhebungen auf.

Diese Maßnahme hat den Vorteil, dass durch die Spitzen der pyramidenartigen Erhebungen zahlreiche, jedoch sanfte Eingriffspunkte an der Außenseite des Herzens vorhanden sind, an die die Haltefinger angelegt sind. Dies ist für das Herz völlig atraumatisch und führt zu einer effektiven Gleithemmung.

In einer weiteren Ausgestaltung der Erfindung weist die pyramidenartige Erhebung die Grundfläche einer Raute auf.

Diese Maßnahme hat den Vorteil, dass eine solche Oberflächenstruktur fertigungstechnisch einfach herzustellen und eine solche Oberflächenstruktur auch einfach zu reinigen und zu sterilisieren ist.

In einer weiteren Ausgestaltung der Erfindung erstreckt sich die längere Diagonale der Raute in Längsrichtung des Haltefingers und die kürzere Diagonale der Raute in Querrichtung des Haltefingers.

Diese Maßnahme hat den Vorteil, dass der Gleithemmeffekt in Querrichtung des Streifens größer ist, da in Querrichtung mehr pyramidenartige Erhebungen pro Längeneinheit vorhanden sind als in Längsrichtung. Dadurch wird insbesondere gehemmt, dass sich das schlagende Herz durch Verdrehung aus dem Korb herausdreht oder in diesem bewegt. Die geringere Gleithemmwirkung in Längsrichtung des Herzens erlaubt die natürlichen geringfügigen axialen Bewegungen des pulsierenden Herzens. Dennoch ist ein sicheres Halten des hochgehaltenen Herzens sichergestellt.

In einer weiteren Ausgestaltung der Erfindung sind vier Haltefinger vorgesehen.

Diese Maßnahme hat den Vorteil, dass bei extrem großen Herzen auf Grund von pathologischen Fehlbildungen dennoch eine sichere Halterung gewährleistet ist, die normalerweise bereits durch drei Finger gewährleistet ist. Ein vierter Finger kann ganz einfach zusätzlich an die Vorrichtung angebracht werden, da ohnehin ein Mechanismus vorhanden ist, der die streifenförmigen Haltefinger auffächern lässt und, meist über eine Feststellschraube, feststellt. Es ist dann möglich, zusätzlich einen vierten Finger für Ausnahmefälle vorzusehen.

In einer weiteren Ausgestaltung der Erfindung weist die Vorrichtung zumindest einen von der Befestigungsvorrichtung abstehenden Arm auf, an dessen äußeren Ende die Haltefinger angeordnet sind.

Diese Maßnahme hat den Vorteil, dass zwischen dem eigentlichen Herzhalter und dem Retraktor über den von der Befestigungsvorrichtung abstehenden Arm eine raumsparende und das Operationsfeld nicht behindernde Verbindung geschaffen ist.

In einer weiteren Ausgestaltung der Erfindung ist der Arm als in allen Raumrichtungen bewegbarer Arm gebildet.

Diese Maßnahme hat den erheblichen Vorteil, dass der Operateur den Arm in einer Raumrichtung ausrichten kann, die sein Aktionsbereich während der Operation oder spätestens bei dem eigentlichen Eingriff am hochgehaltenen Herzen nicht beeinträchtigt.

In einer weiteren Ausgestaltung der Erfindung ist der Arm in einer jeweiligen räumlichen Ausrichtung feststellbar.

Diese Maßnahme hat den Vorteil, dass der Arm nach der entsprechenden Ausrichtung festgestellt bzw. fixiert werden kann. Dies eröffnet auch die Möglichkeit während der Operation, also während von der Vorrichtung das Herz hochgehalten wird, die Ausrichtung des Armes zu verändern, je nachdem wie das die örtlichen Gegebenheiten oder der jeweilige Operationsabschnitt bzw. Verlauf erforderlich macht. Die Art und Weise der Verstellung und der Feststellung des Armes kann durch unterschiedliche Ausgestaltungen realisiert werden. So kann der Arm aus mehreren Gliedern bestehen, die relativ zueinander beweglich sind, und die durch Feststellmechanismen fixiert werden können.

Es versteht sich, dass die vorstehend genannten und nachstehend noch zu erläuternden Maßnahmen nicht nur in den angegebenen Kombinationen sondern auch in anderen Kombinationen oder in Alleinstellung einsetzbar sind ohne den Rahmen der vorliegenden Erfindung zu verlassen.

Die Erfindung wird nachfolgend anhand eines ausgewählten Ausführungsbeispieles im Zusammenhang mit den beiliegenden Zeichnungen näher beschrieben und erläutert.

Es zeigen,
- Fig. 1: eine Seitenansicht, teilweise im Schnitt, eines erfindungsgemäßen Funktionselementes mit einem Herzhalter mit drei Haltefingern,
- Fig. 2: teilweise im Schnitt eine detaillierte Darstellung eines Kugelgelenks wie es beim Arm des Funktionselementes zweimal zum Einsatz kommt,
- Fig. 3: eine stark vergrößerte ausschnittsweise Darstellung eines Verstell- und Feststellmechanismus der drei Haltefinger,
- Fig. 4: eine stark schematisierte vereinfachte perspektive Darstellung des Einsatzes des erfindungsgemäßen Funktionselementes, angebracht an einen Retraktor, zum Halten eines schlagenden Herzens,
- Fig. 5: eine Draufsicht auf einen endseitigen Abschnitt eines Haltefingers mit einer gleithemmenden Oberflächenstruktur,
- Fig. 6a: einen Längsschnitt des Haltefingers von Fig. 5 längs der langen Diagonalen der rautenförmigen Oberflächenstruktur, und
- Fig. 6b: einen Querschnitt des Haltefingers von Fig. 5 längs der kurzen Diagonalen der rautenförmigen Oberflächenstruktur.

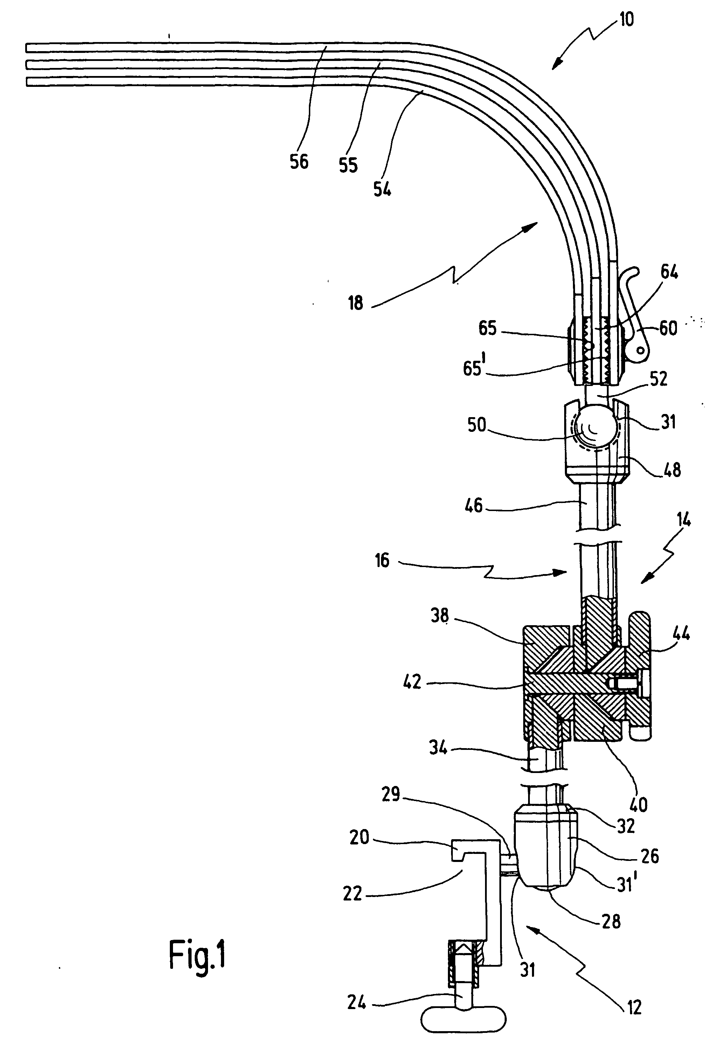

Eine in Fig. 1 dargestelltes Funktionselement ist in seiner Gesamtheit mit der Bezugsziffer 10 bezeichnet.

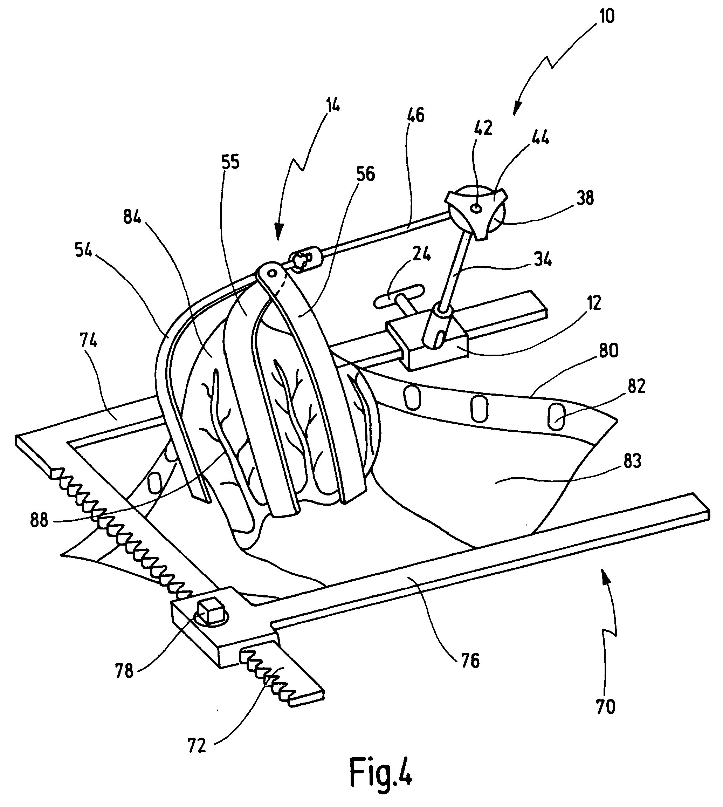

Das Funktionselement 10 weist eine Befestigungsvorrichtung 12 auf, über die es, wie nachfolgend noch im Zusammenhang mit Fig. 4 näher erläutert, an einen Retraktor 70 anbringbar ist.

Das Funktionselement 10 weist ferner eine Vorrichtung 14 zum Halten eines Herzens 84 auf.

Diese Vorrichtung 14 weist einen von der Befestigungsvorrichtung 12 vorspringenden Arm 16 auf, an dessen äußerem Ende der eigentliche Herzhalter 18 angeordnet ist.

Die Befestigungsvorrichtung 12 weist einen Block 20 auf, in dem eine Aussparung 22 vorhanden ist, die der Kontur einer Schiene 74 des Retraktors 70 entspricht, wie dies in Fig. 4 ersichtlich ist. Der Block 20 kann vom äußeren Ende auf die Schiene 74 aufgeschoben werden. Eine Feststellschraube 24 ist dazu vorgesehen, die Befestigungsvorrichtung 24 ortsfest an dem Retraktor 70 zu befestigen.

Der Block 20 ist mit einem ersten Kugelgelenk 26 verbunden.

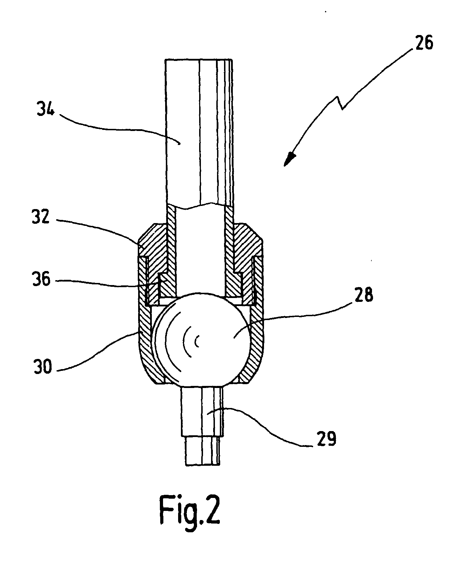

Ein solches Kugelgelenk 26 ist in Fig. 2 in vergrößertem Maßstab im Schnitt dargestellt.

Die Kugel 28 des Kugelgelenkes 26 ist mit einem radial vorspringenden Zapfen 29 verbunden, über den das Kugelgelenk 26 mit der Außenseite des Blocks 20 verbunden ist.

Die Kugel 28 ist in einer Hülse 30 aufgenommen, die diametral gegenüberliegend zwei U-förmige Aussparungen 31 aufweist, wie sie in Fig. 1 beispielsweise an dem oberen zweiten Kugelgelenk 48 ersichtlich ist.

Diese seitlichen Aussparungen 31 ermöglichen ein Verschwenken der Kugel 28 samt Zapfen 29 in der Hülse 30 etwa um 180°.

In die Hülse 30 bzw. in ein nicht näher bezeichnetes Innengewinde ist ein Drehring 32 eingedreht.

Im Drehring ist eine hohle Stange 34 aufgenommen, deren eines Ende der Kugel 28 zugewandt ist, und das mit einem durchmessergrößeren Ringflansch 36 versehen ist.

Der Drehring 32 ruht auf der Stufe bzw. der Schulter des Ringflansches 36.

In Fig. 2 ist eine Situation dargestellt, bei der der Drehring 32 so weit in die Hülse 30 eingedreht ist, dass die Stange 34 auf der Kugel 28 ruht, somit das erste Kugelgelenk 26 festgestellt ist, so dass sich der von der Kugel 28 vorstehende Zapfen 29 in Ausrichtung mit der Stange 34 befindet.

Durch Lösen des Drehringes 32 kann die Kugel 23 verschwenkt werden, beispielsweise um 90°, so dass dann eine Position erreicht ist, wie das bei Fig. 1 beim Kugelgelenk 26 dargestellt ist.

Die von der Hülse 30 vorstehende Stange 34 ist an ihrem gegenüberliegendem Ende mit einer Scheibe 38 verbunden.

Diese Scheibe liegt an einer weiteren Scheibe 40 an, von der seitlich eine weitere Stange 46 vorspringt.

Die beiden Scheiben 38 und 40 sind über eine gemeinsame durchgehende Achse 42 koaxal miteinander verbunden und sind über eine Knebelschraube 44, die mit dem entsprechenden Achszapfen verbunden ist, gegeneinander verklemmbar, also feststellbar.

Wird die Knebelschraube 44 gelöst, können die beiden Scheiben 38 und 40 so gegeneinander verdreht werden, dass die beiden Stangen 34 und 46 aus der in Fig. 1 dargestellten gleichsinnigen Ausrichtung in die eine abgewinkelte Ausrichtung, wie sie in Fig. 4 ersichtlich ist, verschwenkt werden. Durch anschließendes Eindrehen der Knebelschraube 44 werden dann die beiden Scheiben 38 und 40 gegeneinander fixiert.

Am äußeren Ende der Stange 46 ist ein zweites Kugelgelenk 48 angeordnet.

Der von der Kugel 50 des zweiten Kugelgelenks 48 vorstehende Zapfen 52 ist mit dem eigentlichen Herzhalter 18 verbunden.

Der Herzhalter 18 weist drei Haltefinger 54, 55 und 56 auf, die aus gebogenen metallischen flachen Streifen aus geglühtem Chirurgenstahl bestehen. Die drei Haltefinger 54, 55 und 56 sind an ihrem einen Ende jeweils um eine gemeinsame Achse 58 relativ zueinander verschwenkbar. Der entsprechende, hier nicht näher bezeichnete Achszapfen, der durch sämtliche drei Haltefinger 54, 55 und 56 hindurchreicht, ist an einem Ende mit einem Teller 58 versehen, am gegenüberliegenden Ende mit einem Knebelhebel 60. Der mittlere Haltefinger 55 weist einen Körper 64 auf, der beidseits mit einer Verzahnung 65 versehen ist.

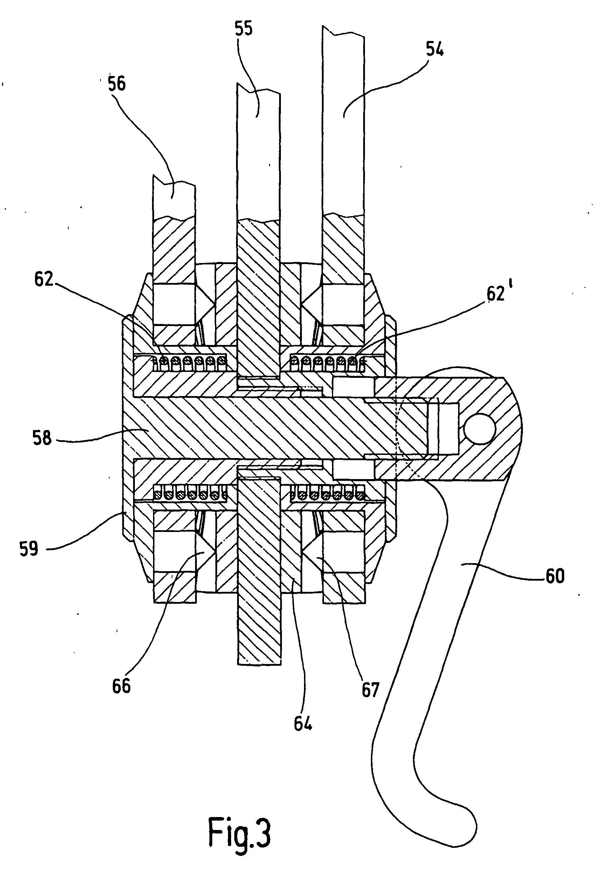

Der, in der Darstellung von Fig. 3 links von dem mittigen Haltefinger 55 angeordnete Haltefinger 56 weist ebenfalls eine Verzahnung 66 auf, die der Verzahnung 65 am Körper 64 entspricht und dieser zugewandt ist.

Dementsprechend weist dann der in der Darstellung von Fig. 3 rechts vom mittigen Haltefinger 55 angeordnete Haltefinger 54 eine entsprechende Verzahnung 67 auf.

Zwischen dem mittigen Haltefinger 55 und dem jeweils benachbarten Haltefinger 54 bzw., 56 ist je eine Schraubenfeder 62 bzw. 62' angeordnet, die jeweils so vorgespannt ist, dass sie den äußeren Haltefinger 54 bzw. 56 von dem mittigen, ortsfesten Haltefinger 55 wegdrückt.

Ist der Knebelhebel 60 ausgeschwenkt, drücken die Federn 62, 62' die Verzahnungen soweit auseinander, dass die drei Haltefinger 54, 55 und 56 relativ zueinander um die Achse 58 verschwenkt werden können. Wird der Knebelhebel 60 eingeschwenkt, werden die drei Bauelemente gegeneinandergedrückt bis die Verzahnungen einrasten, wie das in Fig. 1 dargestellt ist, so dass dann die drei Haltefinger 54, 55 und 56 in der entsprechenden Position fixiert sind, wie beispielsweise in der in Fig. 4 dargestellten aufgefächerten Position.

Der mittige Haltefinger 55 ist fest über seinen Ringkörper 64 mit dem Zapfen 52 des Kugelgelenks 48 verbunden.

Die Arbeitsweise des Funktionselementes 10 soll im Rahmen eines herzchirurgischen Eingriffes, wie er in Fig. 4 schematisch dargestellt ist, näher beschrieben und erläutert werden.

Der Brustkorb 80 eines Patienten wurde längs des Brustbeines aufgetrennt und es wird ein Retraktor 70 angesetzt. An den beiden parallelen, relativ zueinander verschiebbaren Schienen 74 und 76 sind hier nicht gezeigte Valven angebracht, die in den Einschnitt eingeschoben werden. Durch Drehen des Vierkantes 78 mittels eines hier nicht gezeigten Drehhebels werden die Schienen 74 und 76 voneinander wegbewegt und dabei wird der Brustkorb 80 gespreizt, wie dies in Fig. 4 dargestellt ist. Dabei werden auch die Rippen 82 seitlich gespreizt, so dass dann eine Öffnung 83 im Brustkorb 80 vorhanden ist, über die Zugang zu dem auf einer Seite des Brustkorb 80 liegenden Herzens 84 möglich ist.

Um an einer Herzhinterwand 86 einen chirurgischen Eingriff vornehmen zu können, beispielsweise an einem Herzkranzgefäß 88, wird das Herz 84 im Bereich dessen Spitze aus dem Brustkorb 80 angehoben und mittels des erfindungsgemäßen Funktionselementes 10 in dieser Position, wie sie in Fig. 4 dargestellt ist, gehalten. Das Herz 84 schlägt dabei und ist nach wie vor mit den entsprechenden Blutgefäßen verbunden.

Das erfindungsgemäße Funktionselement 10 wird über dessen Befestigungsvorrichtung 12 auf die Schiene 74 aufgeschoben, in eine geeignete Position gebracht und der Block 20 über die Feststellschraube 24 fixiert. Anschließend wird durch entsprechende Manipulationen an der Knebelschraube 44 die räumliche Ausrichtung des Armes 16 in eine günstige Position bewerkstelligt, beispielsweise in die in Fig. 4 dargestellte abgewinkelte Position der beiden Stangen 34 und 46. Nunmehr wird der Knebelhebel 60 gelöst, so dass dann die bislang etwa deckungsgleich übereinanderliegenden Haltefinger 54, 55 und 56 fächerartig aufgespreizt werden können und zwar derart, dass zwischen diesen die Spitze des angehobenen Herzens 84 aufgenommen und gehalten wird. Dazu hebt der Operateur das schlagende Herz 84 an, legt die drei Haltefinger 54, 55 und 56 entsprechend günstig an. Auf Grund der Verformbarkeit können die Haltefinger 54, 55 und 56 jeweils einzeln in eine jeweils günstige Form gebogen werden, so dass letztendlich ein Haltekorb entsteht, in dem das schlagende Herz 84 in der angehobenen Position hochgehalten wird. Anschließend arretiert der Operateur die Stellung über den Kniehebel 60. Dabei positioniert der Operateur das Herz 84 so, dass er beispielsweise zu einem Gefäß 88 der Herzhinterwand 86 an dem er einen operativen Eingriff vornehmen möchte guten Zugang hat. Bei der Manipulation an dem Gefäß 88 werden weitere Funktionselemente zu Hilfe genommen, z. B. ein OPCAB (=Off Pump Coronary Artery Bypass)-Stabilisator oder ein MIDCAB-Sperrer. In der in Fig. 4 dargestellten Ausrichtung des Funktionselementes 10 hält dieses das hochgehobene Herz 84 ortsfest und ermöglicht dennoch ein pulsierendes rhythmisches Schlagen des in dem Herzhalter 18 gehaltenen Herzens 84.

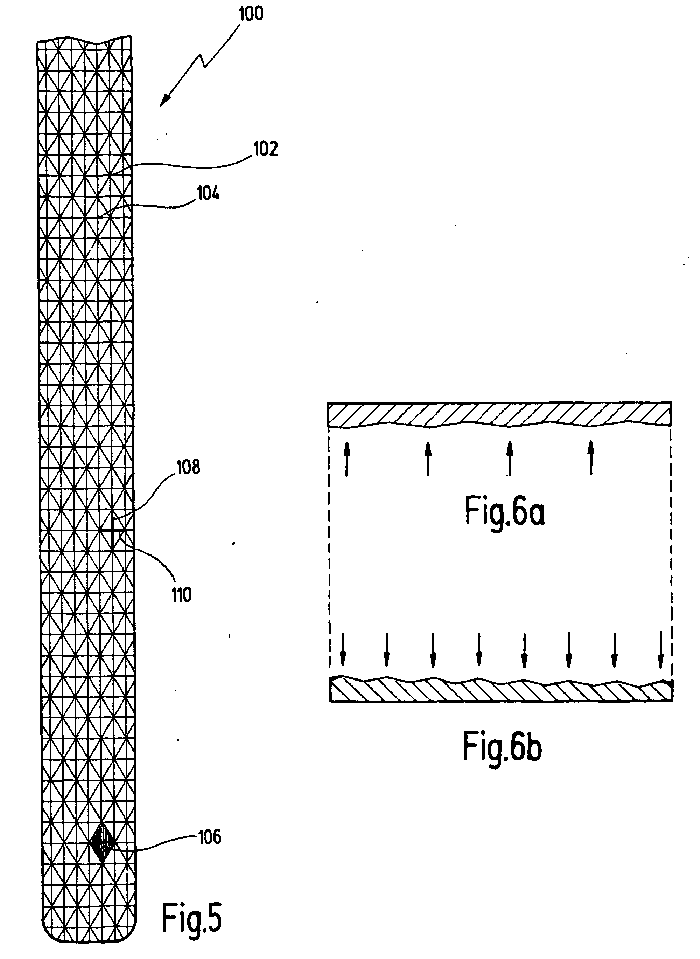

In den Fig. 5 und 6 ist dargestellt, dass ein Haltefinger 100 auf der Seite, die dem Herzen 84 zugewandt ist, mit einer gleithemmenden Oberflächenstruktur 102 versehen ist. Diese gleithemmende Oberflächenstruktur 102 kann selbstverständlich auch auf den zuvor beschriebenen Haltefingern 54, 55 und 56 vorgesehen sein.

Die gleithemmende Oberflächenstruktur 102 besteht aus einer Reihe an zweidimensional aneinander gereihten pyramidenartigen Erhebungen 104. Jede Pyramide weist die Grundfläche einer Raute 106 auf. Die pyramidenartigen Erhebungen 104 sind so aneinander gereiht, dass in Längsrichtung des Haltefingers 102 gesehen die Rauten 106 der pyramidenartigen Erhebungen 104 längs ihrer längeren Diagonale 108 aneinander gereiht sind.

In Querrichtung sind die Rauten 106 jeweils längs ihrer kürzeren Diagonale 110 aneinander gereiht.

Dadurch entsteht eine Oberflächenstruktur 102, die in Längsrichtung etwas geringer gleithemmend ist als in Querrichtung des Streifens 100.

Fig. 6a zeigt einen Längsschnitt des Streifens 100 entlang der längeren Diagonale 108 gesehen und zwar über eine bestimmte Längeneinheit. Dabei ist zu erkennen, dass über diese Längeneinheit vier Spitzen der pyramidenartigen Erhebungen vorhanden sind.

Fig. 6b zeigt einen Querschnitt längs der kürzeren Diagonale 110 über dieselbe Längeneinheit, woraus ersichtlich ist, dass hier acht Spitzen der pyramidenartigen Erhebungen vorhanden sind, wie das jeweils durch Pfeile angedeutet ist. Diese erhöhte Gleithemmung in Querrichtung sorgt dafür, dass sich das Herz 84 in einem aus drei Streifen gebildeten Korb nicht dreht, dennoch aber eine gewisse Längsbewegung, die beim Schlagen des Herzens erfolgt, weniger stark gehemmt ist.

Nach Beendigung des chirurgischen Eingriffes, werden dann die Haltefinger 54, 55 und 56 bzw. 100 wieder gelöst, das Herz 84 in den Brustkorb 80 zurückgelegt und anschließend der Brustkorb verschlossen.

## Patentansprüche

1. Funktionselement zum Anbringen an einen Retraktor (70) zur Durchführung von herz- und thoraxchirurgischen Eingriffen, mit einer Befestigungsvorrichtung (12) zum lösbaren Anbringen an den Retraktor (70), mit einer Vorrichtung (14) zum Hochhalten eines von einer operationsstelle angehobenen schlagenden Herzens (84), wobei die Vorrichtung mehrere spreizbare streifenförmige Haltefinger aufweist, **dadurch gekennzeichnet, dass** drei oder vier ausschließlich streifenförmige Haltefinger (54, 55, 56; 100) vorhanden sind, die derart biegsam ausgebildet sind, dass diese zu einem an das jeweils zu haltende Herz (84) angepassten Haltekorb formbar sind.

2. Funktionselement nach Anspruch 1, **dadurch gekennzeichnet, dass** die Haltefinger (54, 55, 56; 100) in einer bestimmten Schwenkposition feststellbar sind.

3. Funktionselement nach Anspruch 1 oder 2, **dadurch gekennzeichnet, dass** auf der dem Herzen (84) zugewandten Seite eines Haltefingers (100) eine gleithemmende Oberflächenstruktur (102) vorhanden ist.

4. Funktionselement nach Anspruch 3, **dadurch gekennzeichnet, dass** die Oberflächenstruktur (102) pyramidenartige Erhebungen (104) aufweist.

5. Funktionselement nach Anspruch 4, **dadurch gekennzeichnet, dass** eine pyramidenartige Erhebung (104) die Grundfläche einer Raute (106) aufweist.

6. Funktionselement nach Anspruch 5, **dadurch gekennzeichnet, dass** sich die längere Diagonale (108) der Raute (106) in Längsrichtung des Haltefingers (100) und die kürzere Diagonale (110) der Raute (106) in Querrichtung des Haltefingers (100) erstreckt.

7. Funktionselement nach einem der Ansprüche 1 bis 6, **dadurch gekennzeichnet, dass** die Vorrichtung (14) zumindest einen von der Befestigungsvorrichtung (12) abstehenden Arm (16) aufweist, an dessen äußerem Ende die Haltefinger (54, 55, 56; 100) angeordnet sind.

8. Funktionselement nach Anspruch 7, **dadurch gekennzeichnet, dass** der Arm (16) als in allen Raumrichtungen bewegbarer Arm (16) ausgebildet ist.

9. Funktionselement nach Anspruch 8, **dadurch gekennzeichnet, dass** der Arm (16) in einer jeweiligen räumlichen Ausrichtung feststellbar ist.

## Claims

1. Functional element to be mounted on a retractor (70) for performing heart and thorax surgery, with a securing device (12) for mounting it releasably on the retractor (70), and with a device (14) for holding up a beating heart (84) lifted from an operating site, said device having several spreadable strip-shaped holding fingers, **characterized in that** three or four solely strip-shaped holding fingers (54, 55, 56; 100) are present, which are designed to be bendable, so that they can be shaped to form a holding basket adapted to the particular heart (84) which is to be held.

2. Functional element of claim 1, **characterized in that** the holding fingers (54, 55, 56; 100) can be locked in a defined position of pivoting.

3. Functional element of claims 1 or 2, **characterized in that** a slide-inhibiting surface structure (102) is present on that side of a holding finger (100) facing towards the heart (84).

4. Functional element of claim 3, **characterized in that** the surface structure (102) has pyramid-shaped elevations (104).

5. Functional element of claim 4, **characterized in that** a pyramid-shaped elevation (104) has the base surface of a rhombus (106).

6. Functional element of claim 5, **characterized in that** the longer diagonal (108) of the rhombus (106) extends in the longitudinal direction of the holding finger (100), and the shorter diagonal (110) of the rhombus (106) extends in the transverse direction of the holding finger (100).

7. Functional element of anyone of claims 1 through 6, **characterized in that** the device (14) has at least one arm (16) which protrudes from the securing device (12), and at whose outer end the holding fingers (54, 55, 56; 100) are arranged.

8. Functional element of claim 7, **characterized in that** the arm (16) is designed as an arm (16) which can be moved in all directions in space.

9. Functional element of claim 8, **characterized in that** the arm (16) can be locked in a particular spatial orientation.

## Revendications

1. Élément fonctionnel à monter sur un rétracteur (70) en vue de la mise en oeuvre des interventions en chirurgie cardiaque et thoracique, comportant un dispositif de fixation (12) pour le montage amovible sur le rétracteur (70), un dispositif (14) destiné à maintenir un coeur battant (84) en position relevée pour dégager un site opératoire, le dispositif comportant plusieurs doigts de retenue en forme de lames pouvant être écartés, **caractérisé en ce qu'**il est prévu trois ou quatre doigts de retenue (54, 55, 56 ; 100) exclusivement en forme de lames, qui sont flexibles au point de pouvoir les déformer pour obtenir un panier de retenue adapté dans chaque cas au coeur (84) à retenir.

2. Élément fonctionnel selon la revendication 1, **caractérisé en ce que** les doigts de retenue (54, 55, 56 ; 100) peuvent être bloqués dans une position de pivotement déterminée.

3. Élément fonctionnel selon la revendication 1 ou 2, **caractérisé en ce que**, sur le côté, orienté vers le coeur (84), d'un doigt de retenue (100), il est prévu une structure superficielle (102) anti-glissement.

4. Élément fonctionnel selon la revendication 3, **caractérisé en ce que** la structure superficielle (102) comporte des saillies (104) en forme de pyramide.

5. Élément fonctionnel selon la revendication 4, **caractérisé en ce qu'**une saillie (104) pyramidale a une surface de base en forme de losange (106).

6. Élément fonctionnel selon la revendication 5, **caractérisé en ce que** la plus grande diagonale (108) du losange (106) s'étend dans le sens longitudinal du doigt de retenue (100) et la plus petite diagonale (110) du losange (106) s'étant dans le sens transversal du doigt de retenue (100).

7. Élément fonctionnel selon l'une quelconque des revendications 1 à 6, **caractérisé en ce que** le dispositif (14) comporte au moins un bras (16), en porte-à-faux sur le dispositif de fixation (12) et sur l'extrémité extérieure duquel sont agencés les doigts de retenue (54, 55, 56 ; 100).

8. Élément fonctionnel selon la revendication 7, **caractérisé en ce que** le bras (16) est conçu sous forme de bras (16) mobile dans toutes les directions dans l'espace.

9. Élément fonctionnel selon la revendication 8, **caractérisé en ce que** le bras (16) peut être bloqué dans une orientation dans l'espace particulière.
